# EUROPEAN PATENT SPECIFICATION

(11) **EP 2 043 716 B1**
(45) Date of publication and mention of the grant of the patent: **04.08.2010**
(21) Application number: 07732781.5
(22) Date of filing: 11.05.2007
(51) Int. Cl.: A61M 15/00

(54) **A SIMPLE INHALER**
EINFACHER INHALATOR
SIMPLE INHALATEUR

(30) Priority: 16.05.2006 PT 10348106
(43) Date of publication of application: 08.04.2009
(73) Proprietor: Hovione Inter AG, 6000 Lucerne 7 (CH)
(72) Inventor: VILLAX, Peter, 1200-671 Lisbon (PT); MCDERMENT, Iain, Hertfordshire SG8 6EE (GB); BUNCE, Martin, Wiltshire SN9 5JG (GB)
(74) Representative: Hedges, Martin Nicholas
(86) International application number: PCT/GB2007/001756
(87) International publication number: WO 2007/132217

(56) References cited:
- EP-A- 1 504 781
- WO-A-99/36116
- US-A- 5 301 666

## Description

The present invention describes a pulmonary or nasal inhaler of simple construction and operation.

Inhalers used for the delivery of pharmaceutical compounds are widely known, becoming widespread after the development of the dosing valve and pressurized metered dose inhaler by Charles Thiel in 1956, and the introduction of several dry powder inhalers, which started in the 1960s and continues to this day. These inhalers have been used chiefly in the treatment of diseases such as asthma and chronic obstructive pulmonary disease, but recently applications have been developed to deliver drugs systemically via the lung or nose.

The quest to combine efficacy, ease of use, convenience and small size have dominated these efforts. Early devices made use of capsules (GB 1,182,779, Spinhaler; GB 2 064 336, Rotahaler; US 4,859,114, Inhalator; and FR 75 21844, Cyclohaler), but capsules require dexterity in handling, which is a matter of inconvenience, and adds to the cost. Many also possess a cutting or piercing or opening mechanism, in most cases necessitating the use of metal needles or blades (PT 101.450 FlowCaps), yet another source of cost. Ways of avoiding capsule handling can be found in US 5,595,175 and US 5,651,359, but the method by which this has been achieved has brought more mechanical complexity and therefore higher cost.

In situations where an infectious agent is being treated or is simply present in the mouth and airways, there is the need to eliminate the possibility of inhaler contamination and to this end it is highly advantageous to have a sufficiently economic device so that it may be used once and disposed of. Indeed, inhalers to treat viral diseases such as influenza effectively are known (GB 2 178 965 Diskhaler), but the inhaler requires to be re-loaded and re-used over the entire length of the five-day treatment, being repeatedly contaminated with the virus. Moreover, the great majority of influenza patients are inhaler-naive, requiring that the device be of extreme simplicity and intuitive to use.

Consequently, significant attention has been given to disposable devices, and very economical designs have appeared in the literature. A recurring challenge for inventors has been the need to segregate the powder dose so as to prevent it from spilling out prior to use. In one design, no less than six patents (US 5,533,505, US 5,660,169, US 5,918,594, US 6,102,035, US 6,105,574 and US 6,286,507) have been granted for the same device, describing various mechanisms by which the powder dose might be packaged inside the device. This is not difficult in itself, but is a source of cost and industrial or operational complexity, all factors to be avoided.

US 7,032,593 describes also a simple device containing a dose of powder, which is protected against leakage by means of a tether or film strip which is removed immediately prior to use; but in this and in another embodiment in the same patent, the very fine and freely flowing powder does not appear to be prevented from flowing out through the ventilation holes of the device, immediately prior to inhalation.

Moreover, disposable devices have often failed to address the inhaler's most important function, which is to disperse agglomerates of drug particles and excipients down to their original, inhalable size of less than 5 µm. One of the simplest designs of all uses a simple straw (US 5,797,392, DirectHaler), but the fact that the dispersion and entrainment of the dose occur simultaneously in a very short period of time - a fraction of one second - may reduce the efficiency of the device and result in a lower dose being deposited in the lung. Other very simple devices without apparent powder dispersion features include US 5,042,472, US 5,239,991 and US 6,098,619. A lower efficiency may dictate the need to increase the drug dose to achieve the desired therapeutical effect. In addition, fast delivery means that the entire dose is delivered suddenly and this may cause the "powdery mouth" effect. Neither of these characteristics are desirable.

While asthma inhalers were typically designed to hold a large number of pre-metered or device-metered doses of potent drugs, they were largely unsuited for the delivery of large doses. Pre-metered devices are also to be preferred, as device-dispensed doses have been prone to dose metering variability. Devices using a cup as a metering device included in a sliding mechanism are known (US 4,524,769 Turbuhaler, US 5,575,280 Clickhaler; US 5,829,434 Twisthaler, US 6,332,461 Easyhaler) but they are unsuited to metering large doses, and their function is to measure and transport a dose from a bulk powder reservoir to the mouthpiece channel.

Document WO 99/36116 is considered as the closest prior art.

There is therefore a need for an inhaler that is pre-filled with unit doses of powder, for patient convenience; disposable, for reasons of safety and hygiene; simple, for economic reasons and ease of use; and with a high dispersive and entrainment efficacy, for therapeutic benefit.

The present invention is directed to a dry powder inhaler which seeks to combine all of these characteristics and advantages.

According to the present invention there is provided a dry powder inhaler suitable for pulmonary or nasal delivery, comprising: (a) an inhaler body having a mouthpiece, an opening in the body, and a body front inlet extending from the mouthpiece to the opening in the body for providing fluid communication therebetween; and (b) a cartridge movably mounted in the opening in the body and having at least one powder compartment formed therein which incudes a compartment front inlet; the cartridge being movable relative to the inhaler body between at least a first position in which the compartment front inlet of the at least one powder compartment is offset from the body front inlet so as to isolate the contents of the powder compartment from the mouthpiece, and a second position in which the compartment front inlet of the at least one powder compartment is aligned with the body front inlet each of the inhaler body and the cartridge being of unitary construction such that the inhaler is formed of only two separate parts which are moveable relative to each other; and the cartridge being a close tolerance fit, in opening in the body such that in the first relative position of the cartridge the body substantially sealingly engages the front inlet so as to prevent the contents escaping therefrom; **characterized in that** the inhaler body further includes a body rear inlet extending therethrough to the opening in the inhaler body and the or each powder compartment further includes a compartment rear inlet, wherein, in the first position of the cartridge relative to the body, the body substantially sealingly engages the compartment rear inlet so as, in use, to prevent the contents of the compartment from escaping therefrom, and in the second position of the cartridge relative to the body, the compartment rear inlet aligns with the body rear inlet so as to create an air flow path through the compartment to the mouthpiece, such that the compartment forms a dispersion chamber for delivering the contents of the compartment to the mouthpiece.

The dry-powder inhaler of the present invention is intended for pulmonary or nasal delivery, and includes an inhaler body composed of a mouthpiece or nosepiece and an opening in the body. The inhaler has a body front inlet which allows fluid communication between the mouthpiece and the opening in the body and also includes a powder cartridge mounted in the opening of the inhaler body. The powder cartridge has at least one powder compartment and it is designed to move inside the opening, the inhaler body preferably having means to hold the cartridge in place in the opening and means to limit the amount of travel the cartridge can move inside the opening. Each powder compartment has a compartment front inlet and the cartridge can move inside the opening from a first position, in which the compartment front inlet is offset from the mouthpiece passage, to a second position in which the compartment front inlet is aligned with it. In the first position, there is no fluid communication and the powder inside a cartridge compartment is isolated from the mouthpiece and cannot flow out. In the second position, the cartridge has been moved to a point where a compartment front outlet is aligned with the mouthpiece, thereby defining a dispersion chamber from which the contents of the powder chamber can be delivered to the mouthpiece. This construction results in considerable economic savings, as there is no need to employ storage chambers which are distinct from dispersion chambers. The combination of two components forming a dispersion chamber is an inventive feature of the present invention. Avoiding the storage of powder in one chamber and its dispersion in another, has other technical advantages, namely that losses in transferring the powder from one chamber to another are also avoided and that the surface area where powder might adhere and fail to be properly dispersed and entrained, is reduced. Another advantage is that the user does not have to handle unit doses.

(In the following description, "proximal" refers to points on the inhaler that are closer to the mouth or nose and "distal" to points that are farther; and references to "mouthpiece" include "nosepiece").

In order for a powder compartment to become a dispersion chamber, air must be admitted to it. In the inhaler of the present invention, the inhaler body further includes a body rear inlet, which extends from the inhaler body to the body opening and each powder compartment includes a compartment rear inlet. Furthermore, we have provided the inhaler body with a body front inlet, which is located on the inhaler body, at the distal end of the mouthpiece channel, and with body side inlets, which admit air directly from the atmosphere to the mouthpiece channel and supplement the volume of air crossing the powder compartment. The body side inlets should desirably enter the mouthpiece channel as close as possible to the body front inlet, so as to create turbulence on its surface and avoid any unwanted powder accumulation.

When a powder compartment is in the second position as described above, air is able to travel through the body rear inlet, into the compartment rear inlet, through the compartment, out of it through the compartment front inlet, past the body front inlet, into the mouthpiece channel and finally out of the inhaler. When this flow is established as a result of suction on the mouthpiece and a dose of a pharmaceutical powder is contained inside the compartment, the flow of air will induce turbulence leading to a break up of powder agglomerates and to a dispersion of the particles, for entrainment and final deposition in the intended site, in lung or in the nasal cavity, depending on the application.

This inhaler does not use conventional pharmaceutical capsules, such as gelatine or cellulose/HPMC capsules. Rather, the powder doses are contained inside compartments which are shaped like a capsule. Although other shapes are possible, the rounded extremities of a capsule and its cylindrical, constant section make this shape highly suited to minimize powder retention during and after inhalation. In fact any shape with an absence of sharp angles, such as spherical, oval, frusto-conical, bi-conical and the like would be equally advantageous.

The powder compartments, of which there can be just one or multiple depending on the application, but of which there are preferably one or two, are built, shaped, drilled or moulded inside a cartridge. Thus one single component, which can be manufactured in a single step if by injection moulding, contains the powder compartment or compartments. Whatever the number of powder compartments, it is understood that they are always isolated from each other and that there is no communication between them. Such a cartridge containing two powder compartments is a further inventive feature of the present invention.

The inhaler body and the powder cartridge can be made of any suitable material for pharmaceutical use, but plastics are to be preferred, and the material used for the cartridge should advantageously be transparent.

Whatever the material chosen, both the inhaler body and the powder cartridge should be compatible with the powder intended to be contained and delivered, so as to minimize degradation of the powder by chemical reaction and to reduce powder retention during storage and delivery. To this end several plastics have been tested, namely polypropylene, Nylon, transparent Nylon, amorphous Nylon, Acetyl (POMP): Ultra form N2320, Polyester (PET), K-resin, Polyethylene (LDPE): Riblene MV10, Acetal and others. Results show the plastic grade must be carefully adjusted to the powder used and no material can be identified as being universally appropriate. Moreover, these materials have varying degrees of transparency, and sometimes a trade-off must be made between transparency and powder adhesion minimization.

The powder cartridge has inlets to allow air to travel into a powder compartment, disperse the powder, entrain it into the mouthpiece and out into the patient's mouth. However, the compartment rear inlet, located on the distal air admission side of a powder compartment needs to be very small, so that immediately after filling with powder and before the cartridge unit is inserted into the inhaler body, the powder does not flow out of the compartment rear inlet under the force of gravity. We prefer to make these rear inlets as a narrow slit, one per powder compartment, though the same function could be achieved with several very small round holes, of a diameter of 1 mm or less. However a slit is easier to manufacture by injection moulding, and we prefer each slit width to be 1 mm or less, as we have found that this dimension can block or substantially hinder the flow of powder out of the compartment.

Whether using a slit or a small hole as a shape for the compartment rear inlet to admit air to the powder compartment, we have found that tapering the walls of the slit or hole in the direction of the compartment further promotes the blocking of the powder. Such a tapering creates a funnel space directly above the compartment rear inlet and we have found that the best taper angle to block the powder is included between 179° and the angle of repose of the powder contained in the powder compartment, and preferably between 120% and the angle of repose of the powder. This funnel blocks powder flow under gravity by promoting the bridging of particles above the funnel and is a further inventive feature of the present invention.

Conversely, the compartment front inlet, situated at the proximal end of the powder compartment needs to be sufficiently wide to allow normal filling and high-speed filling of the powder. Usually, this compartment front inlet will be of the same diameter as the powder compartment itself. This means that during filling the powder cartridge is desirably held in the same orientation, compartment rear inlets pointing down to the ground, and compartment front inlets pointing up.

Immediately after filling a powder compartment or compartments with a unit dose of powder, desirably in a factory automated environment, the powder cartridge must be inserted into the inhaler body, which is preferably built so that the compartment front and rear inlets are in contact with a continuous, smooth surface on the inhaler body and the contact is a close tolerance fit with the front wall and the rear wall of the inhaler body where that contact takes place. In the storage position, when the powder compartment is offset from the body front and rear inlets air inlets and from the mouthpiece channel, the powder unit dose is effectively sealed inside its compartment, as the cartridge is held between the front wall and the rear wall of the inhaler body. This close contact with the walls of the inhaler body prevents the powder from leakage during storage and this is a further inventive feature of the present invention. At this point the inhaler is ready for packing, desirably in a foil or aluminium pouch, or pouch or packaging of any other suitable material and under low or equilibrium humidity conditions.

Another method to ensure that the powder does not leak out of the powder compartments during storage or manipulation is to construct the powder cartridge with protruding circular rims around the powder compartment front inlets. In this construction, the close contact will be between the circular rims of the powder compartments and the inhaler body. This is to be preferred, as the contact surface will be minimized and thus the tolerance of the fit between the cartridge and the inhaler body can even be closer than in the previous construction, but without causing such a level of friction that would require excessive force to push the powder cartridge into position for inhalation.

Moreover, the powder cartridge can be constructed from a softer material, more compressible than that of the inhaler body, so that the circular rims of the powder compartments, when coming into contact with the harder material of the inhaler body will be subjected to a compression force due to the close fit, and slightly change shape, becoming wider as they are compressed, thus offering a greater contact surface area and better protection against powder leakage.

Finally, powder leakage can be further ensured by blocking the powder cartridge inside the inhaler body, so that even if the inhaler is subjected to strong vibration during transport, no powder will be lost. This can be achieved in several ways: either shrink-wrapping the packaging pouch around the inhaler, or inserting the inhaler into a rigid packaging shell, or using an adhesive tape around the inhaler body and powder cartridge or using a locking feature moulded into inhaler body and/or the powder cartridge. The pouch or the adhesive tape methods have the added benefit of being tamper-evident systems, which are useful in once-only use, disposable inhalers such as the present one.

In addition to preventing the powder from leaking under gravity, the inlets on the powder compartment as well as those on the inhaler body play an important role in the function of this inhaler, as their diameter and surface areas and their position determine the aerodynamic profile of the device and this in turn determines the comfort with which the user inhales, and the efficacy of the device.

In the case of the present invention, the source of power for powder dispersion and entrainment is the patient's inspiratory flow and this has to be used as efficiently as possible by the inhaler. Such devices, known as passive inhalers, do not need motors or other complex mechanical features to operate, but on the other hand tend to be dependent on the airflow rate: the higher the flow, the better the dispersion and the entrainment and the higher the lung dose. This is undesirable, as the same patient inhaling a different flow rates will obtain variable doses. Accordingly, the development of an inhaler which reaches close to its maximum efficacy at a relatively low flow rate has been a major goal in inhalation technology.

When a powder compartment is in fluid communication with various inhaler inlets, i.e. when it is the inhalation position, air and powder are able to mix and flow out of the inhaler. The function of the body side inlet or inlets is very important here, supplementing the small amount of whatever air is able to penetrate the powder compartment through the narrow slits or small holes of the compartment rear inlet. These body side inlets allow for the inhalation to be comfortable and for the powder/air ratio to be desirably lean in powder and rich in air, thus maximizing the entrainment capability of the air.

With the provision of the body side inlet, the user can comfortably generate a pressure drop of 4 kPa, which is recognized in the pharmacopoeias as a suitable pressure drop from a user point of view, as this is half of the maximum pressure drop that the intercostal muscles and the diaphragm can generate. In the case of the nose, the pressure drop that can be generated is lower (because the resistance to the passage of air in the nasal cavity is higher) and in this case one only needs to change the diameter and number of body side inlets to regulate the ideal pressure drop for a nasal delivery.

Now if the user can apply a considerable suction to the mouthpiece, it is important to avoid a powder exit that is too rapid. This is the function of the configuration of the compartment rear and front inlets, particularly in the way their dimensions can be varied to regulate the flow of air which enters and leaves the inhaler.

So, in addition to avoiding the leakage of powder under gravity, the slits or holes of the compartment rear inlet restrict the admission of air. By experimentation of different slit lengths, or number of small holes, it is possible to tune the inhaler to deliver one dose of powder in the desired length of time, given a constant flow rate. The duration can thus be varied between about 200 milliseconds to several seconds. However we prefer to regulate the lengths of the slits or number of holes to give the user a powder delivery time which is longer than 0.5 second and preferably up to 2 seconds at a flow rate of 35 L/min⁻¹, so that there is an excess of air to transport the powder, reduce the "powdery mouth" feeling and improve lung deposition. At the stated flow rate, the delivery duration of 2 seconds will result in an inhalation volume of 1.2 litres of air. Since a person inhaling through an inhaler uses about 2 litres of air for a complete inhalation (it can be more), this means that there is still about 0.8 litre of air to chase the powder dose deep into the lung. For nasal applications, it is desirable to have a shorter delivery duration. The size and configuration of the body front inlet also have a regulating effect on airflow. In their absence, powder delivery out of the compartment front inlet, which is very wide, would be far too rapid and result in perhaps insufficient dispersion. Thus the body front inlet acts as further barrier to the path of the powder and is therefore advantageously provided, as it slows down the delivery and offers a final obstacle to the particles, assisting in their dispersion. This barrier is preferably provided in the form of several small perforations right at the entrance of the mouthpiece channel, though other shapes can be used as well. The powder storage chamber is now fully transformed into a dispersion chamber, inside the same compartment, which is now facing the perforated barrier of the front body inlet.

Notwithstanding the preferred construction of several perforated holes, industrial considerations may determine the moulding of one or two longitudinal slits in the inhaler body front inlets instead, but with a surface area identical or similar to that of the multiple perforations.

The combination of a restricted flow of air at the air admission side of the compartment and the restrictions at its exit caused by the inhaler body inlet, causes the inhalation flow to be more prolonged and in a delivery desirably more gradual, reducing the "powdery mouth" effect and improving pulmonary deposition. This combination of small holes in the dispersion chamber and an additional inlet downstream of the dispersion chamber have been described in PT 101,450 and are incorporated herein by reference.

With this aerodynamic profile, the compartment is an efficient dispersion chamber. Particles may become further dispersed down the mouthpiece inhalation channel, but the highly turbulent and gradual dispersion of the powder is actually observable inside the compartment, when this is manufactured of a transparent material. A storage chamber which becomes a dispersion chamber, the dispersion chamber being defined by elements situated on different mechanical components which are combined at the time of inhalation, is a further inventive feature of the present invention.

The holes that admit air into the inhaler body from the atmosphere, such as the body rear inlet and body side inlet should preferably be protected against inadvertent blocking, as could easily happen if covered with the patient's finger. We prefer to construct these holes with means to prevent such blockage. For instance, the perimeter of the holes can have crenellations, so that even if covered by a finger, air will be able to flow through the gaps. Other constructions are possible, such as features in the inhaler components which result in air gaps that are narrower than a finger but longer than one fingerprint, so that blocking of air is impossible. Means to prevent the blocking of the admission of air to the inhaler body are an inventive feature of the present application.

When the patient wishes to use the inhaler of the present invention, he or she removes it from its packaging. The container is now in its storage position. The patient now moves the container to the first inhaler position, and at this point the compartment has its rear inlet aligned with the body rear inlet, that is the powder could again leak under the force of gravity. The slits or small holes have thus a further function, as they prevent the powder from leaking under gravity, which would be obviously undesirable. The patient inhales the first dose, according to the instructions for use. If there is a second compartment, the patient moves it to the inhalation position and inhales a second time, repeating the maneuvre as often as there are compartments.

The inhaler of the present case can have several embodiments and those that are now described all constitute inventive features of the present application.

In a first embodiment, the powder cartridge is a tray which is inserted sideways into the inhaler body and is pushed transversally, so that a powder compartment becomes aligned with the mouthpiece channel and inhalation may take place (the Tray model). If the powder cartridge includes a second powder compartment, the patient continues to advance the cartridge in the same direction until the second compartment is also aligned for inhalation to take place. The direction of the movement of the cartridge can be perpendicular to the longitudinal axis of the inhaler body and of the mouthpiece channel, but it could be at another angle, different from a right angle, provided the longitudinal axis of the powder compartment in movement is substantially parallel to that of the mouthpiece channel.

In another embodiment which is a variation of the first, the powder cartridge is composed of not one, but two separate trays, each tray containing one powder compartment (the Split Tray model). Here, one cartridge can be pushed from one side towards the central inhalation position where it can be inhaled, and then the second cartridge can be pushed from the other side, in a contrary direction, displacing the first cartridge which is now empty, occupying the central inhalation position and being inhaled in turn.

A third embodiment of the present invention (the Shuttle model) combines the advantages of the Tray and Split Tray Models, employing a single cartridge moving bi-directionally. Here, the powder cartridge, containing at least one powder compartment, has the same tray shape, but instead of moving in a single direction, it is moved in a first direction towards the central inhalation position where the first powder compartment can be inhaled, and then in the contrary direction so that the second powder compartment may in turn be inhaled.

In a fourth embodiment of the present invention, the powder cartridge is a cylinder which comprises at least one powder compartment, the or each compartment being parallel to the cylinder (and to each other if multiple compartments are present) and in this case a powder compartment is brought into alignment with the mouthpiece channel by rotating the cylinder (the Cylinder model). Here the plane of rotation of the powder cartridge is substantially perpendicular to the longitudinal axis of the inhaler body and mouthpiece channel.

In a fifth embodiment of the present invention, the powder cartridge is a disk which comprises one, two or more powder compartments radially extending from the centre of the disk and in this case a powder compartment is brought into alignment with the mouthpiece channel by turning the disk-shaped powder cartridge (the Disk model). In this case the plane of rotation of the powder cartridge is substantially parallel to the longitudinal axis of the inhaler body and mouthpiece channel.

In all embodiments, in the initial storage position, the powder unit dose is contained in each of the compartment or compartments and in this position the powder is sealed by the contact with the inhaler body walls. When a cartridge is moved and a powder compartment is in the inhalation position, all inlets of the several embodiments are in fluid communication and the suction applied to the mouthpiece causes the powder to become aerosolized and to become entrained into the mouth or nose. In the case of a nasal application, the inhalation channel will ideally comprise a branching off and two symmetrical ends, shaped to fit the nostrils, thus allowing the dose to be inhaled simultaneously by both nostrils.

In the Tray model, the patient can advantageously be informed that the powder cartridge has been advanced to the inhalation position by provision of a mechanical detent, a clicking sound or the like. In the Shuttle, Split Tray, Cylinder and Disk models, there is no need for a mechanical detent as the devices can be easily built with a mechanical feature on the inhaler body or in the cartridge which blocks the sliding or rotation of the cartridge at the precise point where inhalation will take place, so that the user only has to move the cartridge to a point where it comes to a hard stop. This is a further inventive feature of the present invention and is great importance, particularly in indications where patients are not familiar with inhalation, and need to be successful on their very first attempt to use the inhaler.

Tray, Shuttle, Cylinder and Disk models require at least two components, while the Split Tray model requires at least three components. Inhalers using two components only where a factory metered dose powder is included and held in place without leaking by the disposition of the two components, which can be altered at the time of inhalation to allow fluid communication and inhalation to take place, while still preventing unwanted powder leaking, is a further inventive feature of the present invention.

All five embodiments are easy to use, as a single movement of a powder cartridge is sufficient to bring a powder compartment in alignment with the mouthpiece channel and this is an important advantage for the inhalers of this invention. Achieving operation with a single movement of inhaler parts when it consist of at least two components is an inventive feature of the present application.

All of these five embodiments comprise the inventive features detailed in the present application and the person skilled in the art will be able to apply the same teachings to other inhalers so these descriptions in no way limit the invention to the embodiments described.

In order that the invention may be well understood, there will now be described some embodiments thereof, given by way of example, reference being made to the accompanying drawings, in which:
Figures 1 and 2 are longitudinal sections of a first embodiment of the invention in the form of a Tray model;
Figure 3 is a perspective view of a powder cartridge as used in the Tray model
Figures 4 and 5 are front and rear elevations, respectively, of a powder cartridge as used in the Tray model;
Figures 6, 7 and 8 are plans views of the Tray model in operation;
Figures 9, 10 and 11 are perspectives of section views of the Split Tray model in operation;
Figures 12 is a longitudinal section of a third embodiment of the invention in the form of a Shuttle model;
Figure 13 is a transversal section of the Shuttle model;
Figures 14 and 15 are front and rear elevations, respectively, of the Shuttle model;
Figures 16, 17 and 18 are plan views of the Shuttle model in operation;
Figures 19 and 20 are perspective views of an inhaler body and a powder cartridge respectively, according to a further embodiment known as a Cylinder model;
Figure 21 is a longitudinal section of a still further embodiment of the invention referred to as a Disk model;
Figure 22 is a perspective view of an inhaler body and of a powder cartridge of the Disk model;
Figure 23 is a longitudinal section of a detail of a powder compartment. Referring to the drawings, numbered sequentially after the word "Fig.", like numerals indicate like parts, and each of the five embodiments is identified with series of numbers where the number of hundreds is the number of the embodiment (**1x** to **5xx)** and the equivalent feature in each of the embodiments has the same number **xx.**

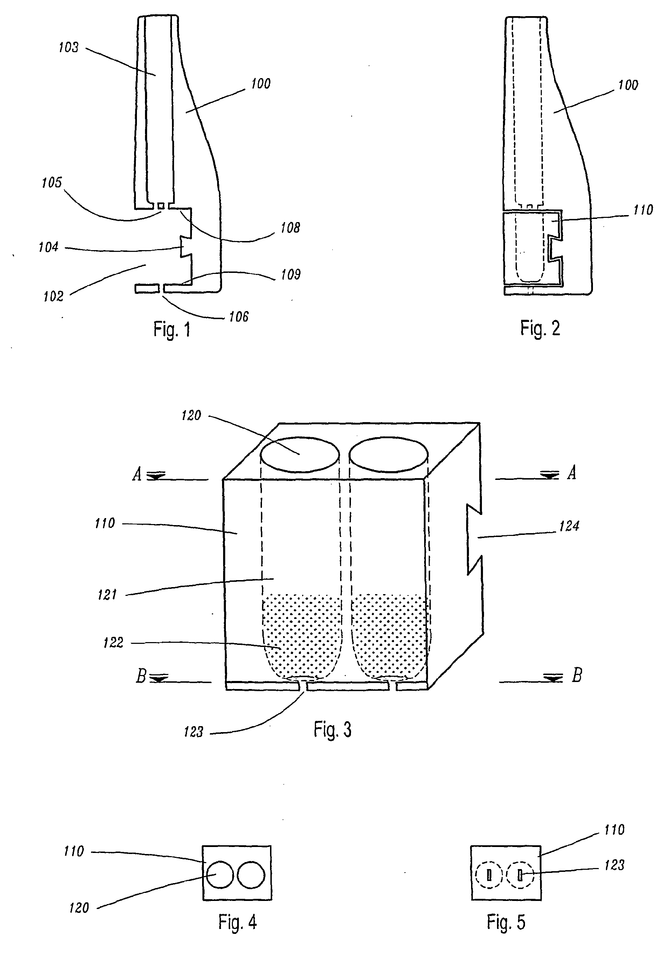

There is shown in Figure 1 a first embodiment of the invention, hereinafter referred to as the Tray model, comprising an inhaler body 100 having an opening 102, a mouthpiece 103, air inlets 105; 106 in the inhaler body, a front wall 108 and a rear wall **109.**

As all the other illustrated embodiments are in many ways similar in construction and operation to the first embodiment, for the sake of clarity not all features are repeated in the drawings, and the expert will have no difficulty in determining where they are required.

As shown in Figure 1, the inhaler body **100** has a rail **104** which guides and holds in place a powder cartridge **110,** shown in more detail in Figure 3, in the manner shown in Figure 2. As can be seen from Figures 2 and 3, the powder cartridge **110** has a rail guide **124** formed on its back surface which is of complementary size and shape to the rail **104,** the interface between the rail and guide restricting the cartridge to a transverse sliding movement across the body opening **102.** As shown in Figure 3, the powder cartridge **110,** used in a Tray model, includes two identical powder compartments **121.** The compartments **121** are shown only partially filled with powder **122,** but they can be filled to full capacity if required, depending upon the characteristics of the powder. Powders with poor flow properties will require free space inside the compartment **121** to become fully aerosolized, whereas free flowing powders are more permeable to air and will become entrained and flow out of the compartment even when it is full. Each compartment **121** is provided in its front face with an compartment front inlet **120,** through which the powder will flow when a suction is applied to mouthpiece **103** with the compartment **121** aligned with body front inlet **105.** In order to enable air to enter the compartment **121** so as to create a through-flow when this suction is applied, each compartment **121** also includes a compartment rear inlet **123** in the rear face of the cartridge **110,** which must be aligned with body rear inlet **106** for fluid communication to be established. More air is supplemented to the inhalation flow through body side inlets **130** and **131,** which extend from the inhaler body **100** to the mouthpiece channel **103,** immediately above the body front inlet **105**.

Figures 4 and 5 show elevations of the powder cartridge **110.** The front elevation in Figure 4 (from section AA in figure 3) displays a front inlet **120** of a compartment, which will adjoin front wall **108** and face the body front inlet **105** in the inhalation position. The rear elevation in Figure 5 (from section BB in figure 3) shows a rear inlet **123** of a compartment, which will adjoin the rear wall 109, each of which admits air into its associated compartment **121** so that the air can pass through the powder contained therein, each compartment rear inlet **123** being shaped as a very narrow slit, to prevent powder from leaking during powder filling. The rail guide **124** is characteristic of the powder cartridge **110** used in a Tray model, and is absent in the powder compartment of Shuttle or Split Tray model embodiments described hereinafter, where the walls of the powder cartridge **110** can be smooth. A detent is provided on one of the walls of the powder cartridge **110** and on the adjoining area of one of the walls of the opening **102** (neither shown) in the Tray model, giving an audible or tactile sign to the user of the inhaler at the precise positions of inhalation. Moreover, models Shuttle, Split-Tray, Cylinder and Disk can be provided with means to prevent the user from overshooting the inhalation position.

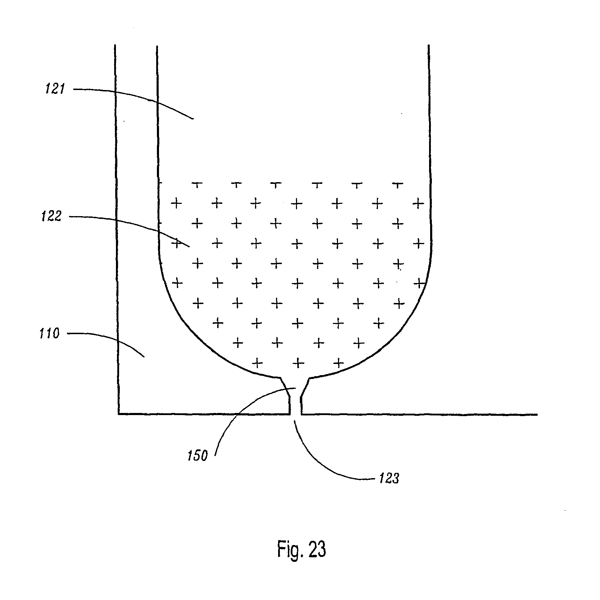

Figure 23 is a detailed section view of a powder cartridge **110** which can be used in the Tray model, but in all other embodiments as well. The walls of compartment rear inlet **123** are parallel at first, but then begin to taper out, to form a funnel **150,** the objective of which is to promote the bridging of the powder **122** above the funnel **150,** or to allow the powder **122** to enter the funnel and to plug it. In both cases the objective is to prevent any leaking of powder out of compartment rear inlet **123.**

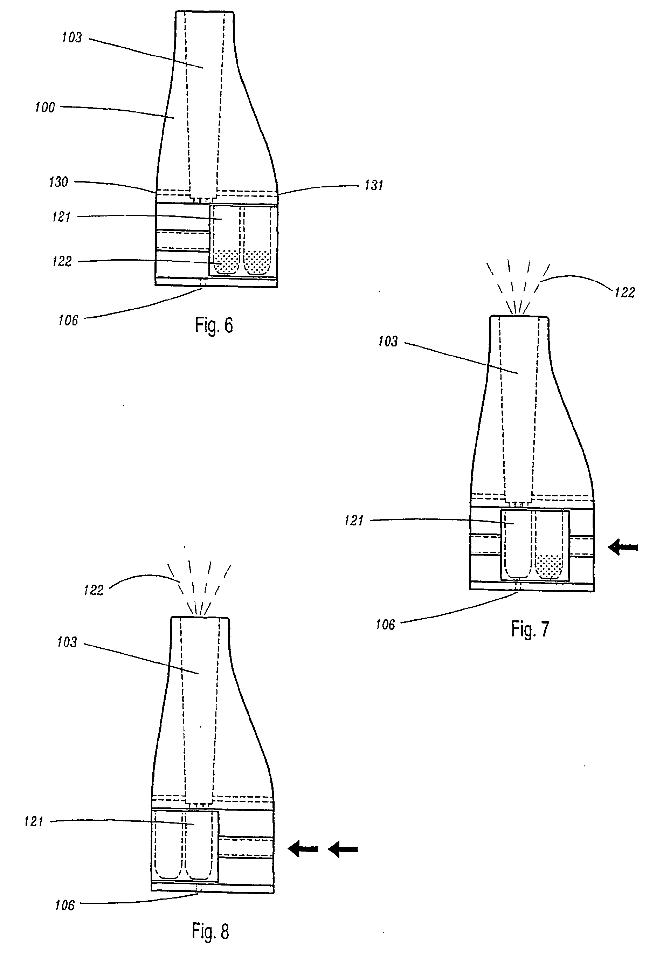

Referring next to Figures 6, 7 and 8, there is shown the Tray model in three different operational configurations. In Figure 6, the powder cartridge **110** is in its storage position with the compartment front inlet **120** of each powder compartment **121** offset from the body front inlet **105** and of the mouthpiece **103** and closed off by the smooth walls **108** and **109** of inhaler body **100** so that the powder **122** is blocked inside the compartment **121.** In Figure 7, the powder cartridge **110** has been advanced from the storage position shown in Figure 6 to a first use position in which one of the powder compartments **121** is aligned with the mouthpiece **103,** establishing fluid communication between body rear inlet **106** and compartment **121,** enabling inhalation of the powder contained therein to take place.

Continued advancement of the cartridge **110** from the first use position shown in Figure 7 brings the cartridge onto a second use position in which the other powder compartment **121** is brought into alignment with mouthpiece **103,** so as to enable inhalation of the powder contained therein to take place.

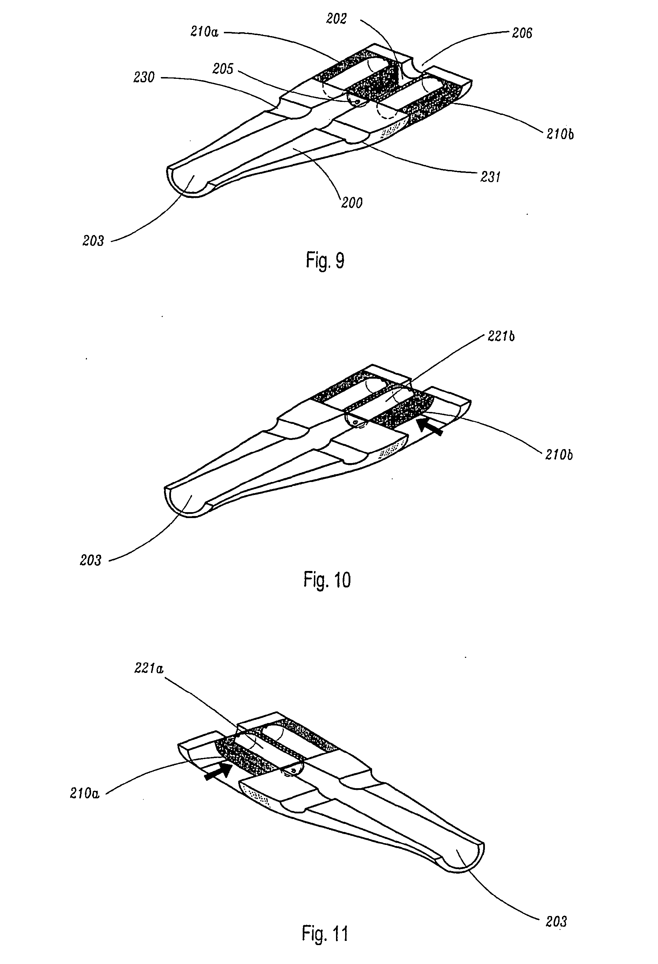

Referring next to Figures 9, 10 and 11, these show a perspective of a longitudinal section of the inhaler body in operation, according to a second embodiment of the invention hereinafter referred to as a Split Tray model. As with the first embodiment, the Split Tray model has an inhaler body **200,** a mouthpiece **203** which extends to body front inlet **205** and which is in communication with body side inlets **230** and **231.** For clarity purposes, the body side inlets have been drawn farther from body front inlet **205** than is desirable. A rear body inlet **206** is again provided at the bottom of the inhaler body **200,** aligned with the longitudinal axis of the mouthpiece **203** and body front inlet **205.**

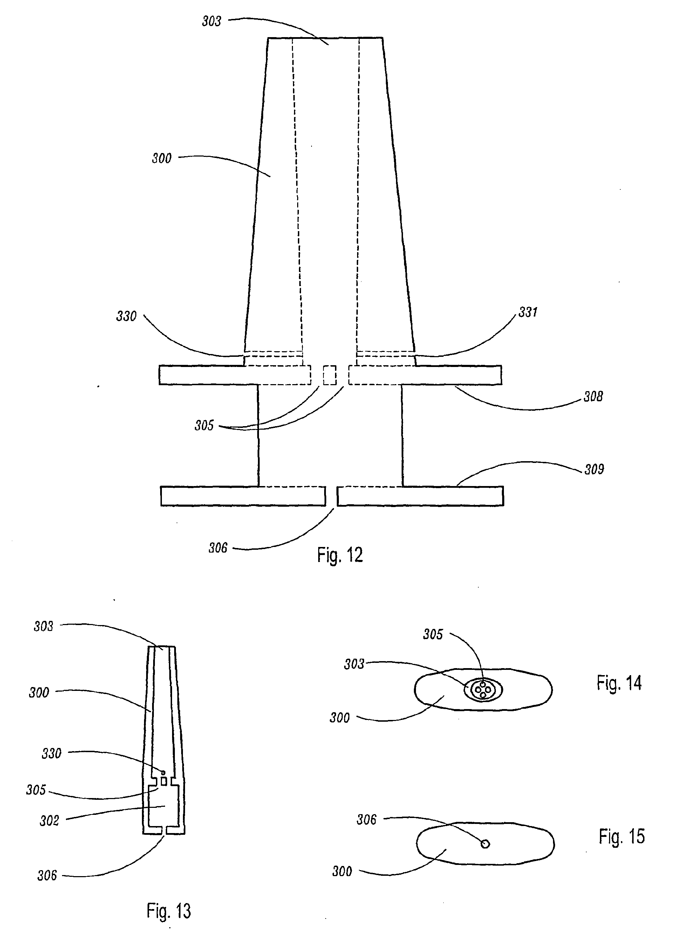

The Split Tray model, unlike the other embodiments, has two powder cartridges **210a, 210b,** which are separately formed from each other, each including just a single compartment. Figure 9 shows the Split tray model in its storage position in which both cartridges are offset from the body front inlet **205** of the mouthpiece - the compartments **221a, 221b** of each cartridge **210a, 210b** being engaged with the smooth walls of inhaler body **200** so that the powder is sealed inside the compartments **221a, 221b.** From the storage position of Figure 9, a first inhaling configuration is achieved by advancing one of the powder cartridges **210b** into the inhaler body **200** as shown in Figure 10 so as to bring the compartment **221b** into alignment with the mouthpiece **203,** thereby enabling inhalation to take place. A second inhaling configuration is then achieved by advancing the other powder cartridge **210a** into the inhaler body **200** in the opposite direction, pushing the now empty powder cartridge **210b** away from the inhalation position and in turn bringing the still powder loaded compartment **221a** of the other cartridge in turn in alignment with the mouthpiece **203.** Inhalation of the powder in the other cartridge can then take place. Referring next to figure 12, there is shown a longitudinal section of the inhaler body according to a third embodiment of the present invention, hereinafter referred to as the Shuttle model. As with the previous embodiments, the Shuttle model has an inhaler body **300,** a mouthpiece **303** which extends to body front inlet **305,** body side inlets **330, 331,** a front wall **308** and a rear wall **309.** A body rear inlet **306** is again provided a the bottom of the inhaler body **300,** aligned with the longitudinal axis of the mouthpiece **303.**

Figure 13 is a transversal section of the Shuttle (or Split Tray) model, which shows more clearly the opening **302** which is designed to hold a powder cartridge. Figure 14 is a front elevation of the inhaler body **300 (200)** used in a Shuttle (or Split Tray) model, showing a mouthpiece **303,** and inside it, a view of the body front inlet **305** which admits air and powder to the mouthpiece **303.** Figure 15 is a rear elevation of the inhaler body **300 (200),** used in the same models, showing here a single body rear inlet **306** to admit air to a powder compartment. As can be seen from these views, the Shuttle model does no include a rail on the body or a rail guide on the cartridge in difference to the first embodiment.

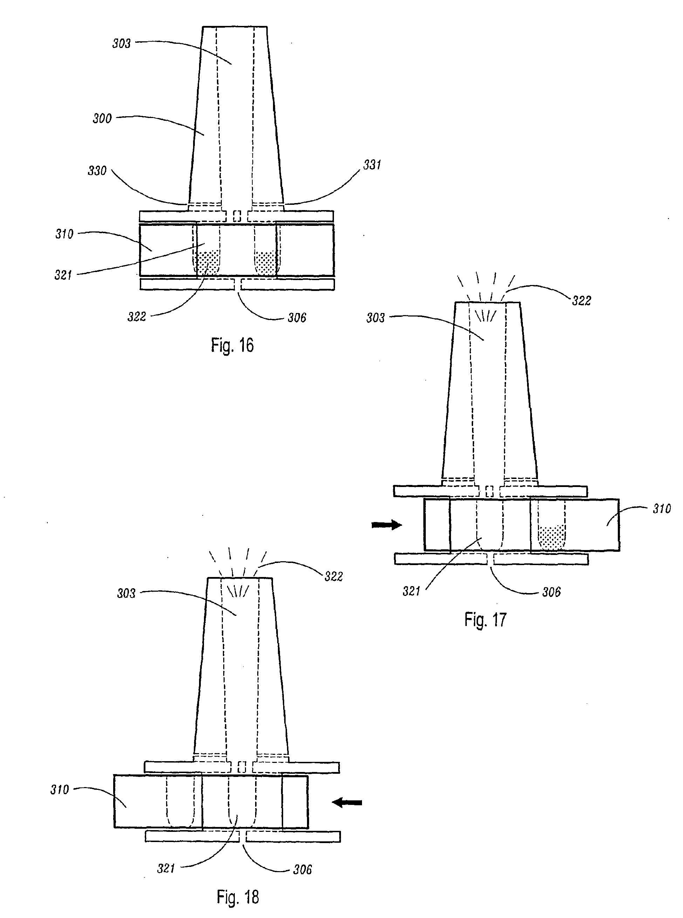

Figures 16, 17 and 18 show the operational positions of the Shuttle model. In Figure 16, the powder cartridge **310** is in its storage position with the powder compartments **321** offset from the mouthpiece **303** and from the body rear inlet **306,** the inlets of compartment **321** instead being sealed off through engagement with the smooth front **308** and rear **309** walls, so that the powder **322** is blocked inside the compartment **321.** In Figure 17, the powder cartridge **310** has been advanced to a first inhaling position in which the powder compartment **321** is aligned with the body rear inlet **306** and with mouthpiece **303,** thereby enabling inhalation of the powder **322** of the first compartment to take place. In Figure 18, the powder cartridge **310** has been advanced in the opposite direction, moving the cartridge back through its storage position and continuing, so that the other compartment **321** is aligned with the mouthpiece **303,** enabling inhalation of the second compartment to take place. Unlike the Tray model, in the case of the Shuttle model the user does not have to take particular care at stopping a powder compartment **321** at a precise inhalation position, and only needs to push the powder cartridge **310** as far as it will go for proper alignment to be achieved.

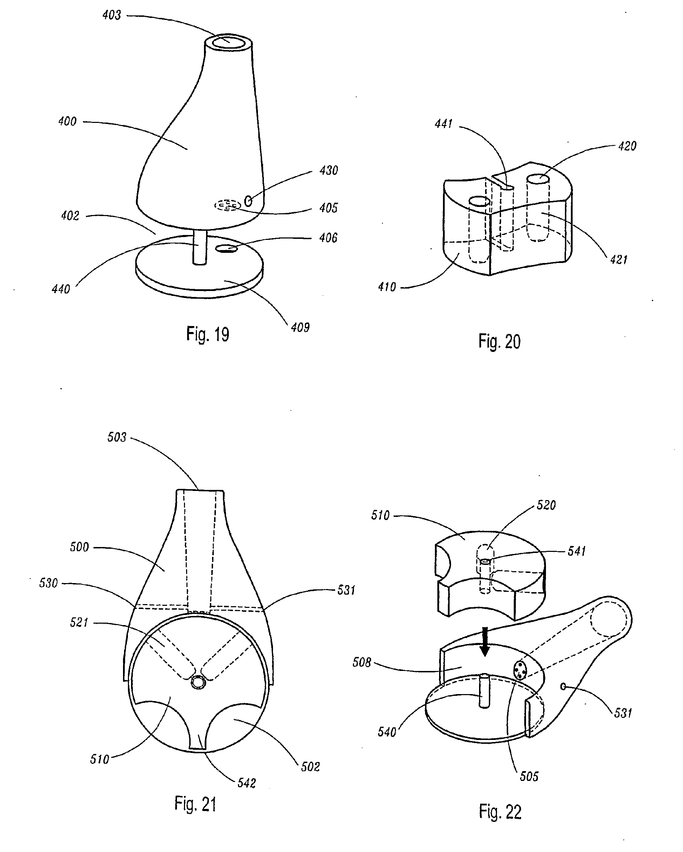

A fourth embodiment is shown in Figures 19 and 20. Figure 19 is a perspective view of a rotary actuated inhaler body which operates in conjunction with a cylinder powder cartridge **410,** which will hereinafter be referred to as the Cylinder model. The difference in relation to the previous embodiments is that the powder cartridge **410** is provided with an opening **441** by means of which it is journal mounted on an inhaler body axle **440** which extends perpendicularly to the longitudinal axis of the mouthpiece so as to enable the cartridge **410** to be rotated relative to the inhaler body **400** in order to bring the powder compartments **420** into inhalation alignment with the mouthpiece **403.** The spindle **440** is hollow and is provided with an inlet (not shown), so that as in all other models, air can flow through each powder compartment **421** and into the mouthpiece **403.** The passage of air through the inhaler is established in substantially the same way as with the Tray, Shuttle and Split Tray models, aligning the compartment front inlet **420** with the mouthpiece channel **403** so as to allow an air flow path through each compartment **421,** and the user turns the powder cartridge **410** first to one side, as far as it will go, to bring one of the compartments into alignment with the mouthpiece **403** and inhale, and then repeat the action in the opposite direction to inhale the powder from the second compartment.

Figure 21 shows a longitudinal section of an inhaler body **500** assembled with a cartridge **510** according to a fifth embodiment of the invention, hereinafter referred to as the Disk model and fig 22 shows a corresponding perspective view. Here the powder cartridge **510** is formed as a disk, the inhaler body opening being a complementary shape to receive the cartridge **510,** and the powder compartments **521** being positioned at a 90° angle in relation to each other. The powder cartridge **510** is further provided with a finger grip **542,** which the user moves from side to side to bring each powder compartment **521** in alignment with the inlet **505** and the mouthpiece **503.**

The operation of the Cylinder and Disk models is analogous to that of the other models, the user rotating the powder cartridge **410; 510** in one direction and then the other, to bring each powder compartment **410; 510** in alignment with mouthpiece **403; 503.** In storage, the powder compartment **421; 521** is prevented from spilling by a close contact with the smooth front and rear walls, of which can be seen rear wall **409** in Figure 19 and front wall **509** in Figure 22. Figure 23 shows a detail of a powder compartment **121,** which can be incorporated into the cartridge **110** of all the above described embodiments. The compartment rear inlet **123** takes the shape of a funnel **150** which helps to promote the bridging of the powder **122** and reduce the probability of leakage during filling.

An inhaler embodiment of the present invention has been tested to determine its aerodynamic profile as well as its powder dose delivery characteristics compared to another marketed dry-powder inhaler, FlowCaps® (Hovione SA, Lisbon, Portugal).

The procedure to measure the pressure drop across the inhaler at a given airflow is described in the European Pharmacopoeia. A Shuttle model prototype was used for this test and the pressure drop across the device was found to be identical to that of FlowCaps, 4.0 kPa when a flow of 35 L/min⁻¹ was applied.

An experimental anti-viral compound was then formulated to determine the dispersion and the entrainment efficacy of both devices. The active ingredient was previously micronized using a conventional jet-mill and particle sizes were achieved where more than 50% of the particles had a diameter smaller than 5 µm, as measured by laser diffraction (Malvern, UK). The active drug was formulated in batches of 1, 5 and 7.5 mg of drug per 25 mg of drug powder blend, where the difference was made up by adding lactose. Two grades of lactose were used, DMV SV003 ("coarse" lactose) and Pharmatose 450M ("fine" lactose), both from DMV (Holland). Instead of lactose, other choices could have been glucose, saccharose, maltose, mannitol, sorbitol, xylitol or dextran, individually or in combination, which are known to be advantageous in powder inhalation formulations.

After blending of the blend components to produce an ordered mix and determining the batch homogeneity, the formulated powder was filled into cellulose capsules, size 4, (Shionogi, Japan) for use with FlowCaps, and into powder compartments, for use with the Shuttle prototype. The inhalers were then tested at a flow rate of 35 L/min⁻¹ on an Andersen cascade impactor (Graseby Andersen, Smyrna, GA), actuated twice to allow a volume of 2 x 2 litres of air to pass through the device, and the mass of active drug deposited at each stage of the cascade impactor was quantified using high pressure liquid chromatography. From these data, the emitted dose and the fine particle dose were calculated, where the emitted dose was the sum of all drug masses collected from each of the impactor stages, including the inductor throat, and the fine particle dose was the mass of drug collected below the 5 µm cutoff point. The ratio of the fine particle dose to the emitted dose is the fine particle fraction and is a measure of inhaler efficiency. The higher the fine particle dose, the higher the lung dose is expected to be. The results are summarized in the following table:

| Nominal dose | | FlowCaps | Shuttle |
|---|---|---|---|
| 1 mg | Emitted dose ED | 0.72 mg | 0.63 mg |
| | Fine particle dose FPD | 0.28 mg | 0.34 mg |
| | Fine particle fraction (ED/FPD) | 38.9% | 54.3% |
| 5 mg | Emitted dose ED | 3.64 mg | 3.18 mg |
| | Fine particle dose FPD | 1.84 mg | 1.92 mg |
| | Fine particle fraction (ED/FPD) | 50.6% | 60.4% |
| 7. 5 mg | Emitted dose ED | 4.24 mg | 5.44 mg |
| | Fine particle dose FPD | 2.30 mg | 2.38 mg |
| | Fine particle fraction (ED/FPD) | 54.2% | 43.8% |

The data indicate that both inhalers have a comparable performance in terms of fine particle dose, correlating well with the particle size of the micronized active drug, and demonstrate that inhalers of the present invention are suitable for the delivery of large doses of pharmaceutical active ingredients, pre-metered directly into the inhaler, without a primary container such as a capsule or a blister. This results in an inhaler which is more economical and simpler to use, without sacrificing performance.

The person skilled in the art will recognize in this performance an ability of the present inhaler to deliver other types of drugs, namely beta2-agonists, anticholinergics, corticosteroids, analgesics, antibiotics, vaccins, proteins, peptides and insulin and other drugs deliverable by inhalation.

## Claims

1. A dry powder inhaler suitable for pulmonary or nasal delivery, comprising:
(a) an inhaler body (100; 300; 400; 500) having a mouthpiece (103; 303; 403; 503), an opening in the body (102; 302; 402; 502), and a body front inlet (105; 305; 405; 505) extending from the mouthpiece to the opening in the body for providing fluid communication therebetween; and
(b) a cartridge (110; 310; 410; 510) movably mounted in the opening in the body and having at least one powder compartment (121; 321; 421; 521) formed therein which includes a compartment front inlet (120; 420; 520);
the cartridge being movable relative to the inhaler body between at least a first position in which the compartment front inlet (120; 420; 520) of the at least one powder compartment (121; 321; 421; 521) is offset from the body front inlet (105; 305; 405; 505) so as to isolate the contents of the powder compartment from the mouthpiece (103; 303; 403; 503), and a second position in which the compartment front inlet (120; 420; 520) of the at least one powder compartment (121; 321; 421; 521) is aligned with the body front inlet (105; 305; 405; 505)
each of the inhaler body (100; 300; 400; 500) and the cartridge (110; 310; 410; 510) being of unitary construction such that the inhaler is formed of only two separate parts which are moveable relative to each other; and
the cartridge (110; 310; 410; 510) being a close tolerance fit in the opening in the body (100; 300; 400; 500) such that in the first relative position of the cartridge (110; 310; 410; 510), the body (100; 300; 400; 500) substantially sealingly engages the front inlet (120; 420; 520) so as to prevent the contents escaping therefrom; **characterized in that**
the inhaler body (100; 300; 400; 500) further includes a body rear inlet (106; 306; 406) extending therethrough to the opening in the inhaler body and the or each powder compartment (121; 321; 421; 521) further includes a compartment rear inlet (123), wherein, in the first position of the cartridge (110; 310; 410; 510) relative to the body (100; 300; 400; 500), the body (100; 300; 400; 500) substantially sealingly engages the compartment rear inlet (123) so as, in use, to prevent the contents of the compartment (121; 321; 421; 521) from escaping therefrom, and in the second position of the cartridge (110; 310; 410; 510) relative to the body (100; 300; 400; 500), the compartment rear inlet (123) aligns with the body rear inlet (106; 306; 406; 506) so as to create an air flow path through the compartment (121; 321; 421; 421) to the mouthpiece (103; 303; 404; 503), such that the compartment (121; 321; 421; 521) forms a dispersion chamber for delivering the contents of the compartment to the mouthpiece (103; 303; 403; 503).

2. A dry powder inhaler according to claim 1, **characterized by** the fact that the compartment rear inlet (123) of the or each powder compartment is sized so as substantially to prevent the powder from passing therethrough out of the compartment (121; 321; 421; 521) under gravity during filling, and the said compartment rear inlet has at least one dimension which is 1 mm or less.

3. A dry powder inhaler according to claim 2, **characterized by** the fact that the or each compartment rear inlet (123), which preferably takes the form of a slit, forms a funnel (150) tapering outwardly towards the compartment.

4. A dry powder inhaler according to claim 3, **characterized by** the fact that the taper angle of the funnel (150) is in the range of between 179° and the angle of repose of the pharmaceutical powder used and preferably between 120% and said angle of repose.

5. A dry powder inhaler according to any of the preceding claims, **characterized by** the fact that the inhaler body (100; 300; 400; 500) further includes means for preventing obstruction of the passage of air through compartment rear inlets (123) and body side inlets (130; 330; 430; 530), by the user.

6. A dry powder inhaler according to any of the preceding claims, **characterized by** the fact that the or each cartridge (110; 310; 410; 510) is a close tolerance fit in the opening in the body (102; 302; 402; 502) such that when the compartment front and rear inlets (120; 420; 520) of the at least one powder compartment (121; 321; 421; 521) are offset from the body front and rear inlets (105; 305; 405; 505), the inhaler body (100; 300; 400; 500) substantially sealingly engages the front (120; 320; 420; 520) and rear (123) compartment inlets so as to prevent powder leaking from said compartment.

7. A dry powder inhaler according to any of the preceding claims, **characterized by** the fact that a body front inlet (105; 305; 405; 505) is provided at the distal end of the mouthpiece (103; 303; 403; 503) (or nosepiece), which body front inlet in combination with a powder compartment (121; 321; 421; 521) defines a powder dispersion area and reduces the flow of air leaving said powder compartment and entering said mouthpiece.

8. A dry powder inhaler according to any of the preceding claims, **characterized by** the fact that the opening in the inhaler body (102; 302) extends transversally of the inhaler body (100; 300) and the cartridge (110; 310) is slidably moveable along the opening (102; 302) transverse to the inhaler body (100; 300), the longitudinal axis of the or each compartment (121; 321) being oriented parallel to the longitudinal axis of the mouthpiece (103; 303).

9. A dry powder inhaler according to any of claims 1 to 7, **characterized by** the fact that the cartridge (410; 510) is rotatably engageable in the opening (402; 502).

10. A dry powder inhaler according to claim 9, **characterized by** the fact that the cartridge (410) is cylindrically shaped and rotates in the opening (402) about an axis (440) parallel to the longitudinal axis of the mouthpiece passage (403).

11. A dry powder inhaler according to claim 9, **characterized by** the fact that the cartridge (510) is disk shaped and rotates in the opening (502) about an axis (540) substantially perpendicular to the longitudinal axis of the mouthpiece passage (503).

12. A dry powder inhaler according to any of the preceding claims, **characterized by** the fact that, in use, the cartridge (110; 310; 410; 510) is advanced to bring a compartment front inlet (120; 420; 520) into alignment with the body front inlet (105; 305; 405; 505) with a single movement of the cartridge.

13. A dry powder inhaler according to any of the preceding claims, **characterized by** the fact that the cartridge (110; 310; 410; 510) includes at least two powder compartments (121; 321; 421; 521) which are isolated from each other.

14. A dry powder inhaler according to claim 13, **characterized by** the fact that it further includes blocking means operable to prevent movement of the cartridge (310; 410; 510) in a first direction beyond the precise point where the first compartment front inlet (420; 520) is in alignment with the body front inlet (305; 405; 505), and similar blocking operable to prevent movement of said cartridge in a direction contrary to the first direction beyond the precise point where the second compartment front inlet (420; 520) is in alignment with said body front inlet.

15. A dry powder inhaler according to any of the preceding claims **characterized by** the fact that it includes just a single cartridge (110; 310; 410; 510), where each of the inhaler body (100; 300; 400; 500) and said cartridge are formed as a unitary part, such that the inhaler is composed of just two parts.

## Patentansprüche

1. Trockenpulver-Inhalator, der sich für die pulmonale oder nasale Verabreichung eignet, enthaltend:
(a) einen Inhalatorkörper (100; 300; 400; 500), der ein Mundstück (103; 303; 403; 503), eine Körperöffnung (102; 302; 402; 502) und einen vorderen Körpereinlass (105; 305; 405; 505) hat, der sich von dem Mundstück zu der Öffnung in dem Körper erstreckt, um zwischen beiden eine Fluidverbindung herzustellen; und
(b) einen Behälter (110; 310; 410; 510), der beweglich in der Öffnung in dem Körper angebracht ist und in dem wenigstens ein Pulverabteil (121; 321; 421; 521) ausgebildet ist, das einen vorderen Abteileinlass (120; 420; 520) hat;
wobei der Behälter relativ zum Inhalatorkörper zwischen wenigstens einer ersten Stellung, in der der vordere Abteileinlass (120; 420; 520) des wenigstens einen Pulverabteils (121; 321; 421; 521) von dem vorderen Körpereinlass (105; 305; 405; 505) derart versetzt ist, dass die Inhalte des Pulverabteils von dem Mundstück (103; 303; 403; 503) abgetrennt sind, und einer zweiten Stellung beweglich ist, in der der vordere Abteileinlass (120; 420; 520) des wenigstens einen Pulverabteils (121; 321; 421; 521) mit dem vorderen Körpereinlass (105; 305; 405; 505) ausgerichtet ist;
sowohl der Inhalatorkörper (100; 300; 400; 500) als auch der Behälter (110; 310; 410; 510) einen derart einheitlichen Aufbau haben, dass der Inhalator aus lediglich zwei getrennten Teilen besteht, die im Bezug zueinander beweglich sind; und
der Behälter (110; 310; 410; 510) mit geringer Toleranz in die Öffnung in dem Körper (100; 300; 400; 500) derart eingepasst ist, dass in der ersten Relativstellung des Behälters (110; 310; 410; 510) der Körper (100; 300; 400; 500) im wesentlichen dichtend mit dem vorderen Einlass (120; 420; 520) in Eingriff steht, um so zu verhindern, dass die Inhalte aus diesem entweichen; **dadurch gekennzeichnet, dass:**
der Inhalatorkörper (100; 300; 400; 500) weiterhin einen hinteren Körpereinlass (106; 306; 406), der sich durch diesen zur Öffnung in dem Inhalatorkörper erstreckt, und das eine oder jedes Pulverabteil (121; 321; 421; 521) weiterhin einen hinteren Abteileinlass (123) enthält, wobei in der ersten Stellung des Behälters (110; 310; 410; 510) relativ zu dem Körper (100; 300; 400; 500) der Körper (100; 300; 400; 500) im wesentlichen dichtend mit dem hinteren Abteileinlass (123) derart in Eingriff steht, um so bei Benutzung zu verhindern, dass die Inhalte des Abteils (121; 321; 421; 521) aus diesem entweichen, und in der zweiten Stellung des Behälters (110; 310; 410; 510) relativ zu dem Körper (100; 300; 400; 500) der hintere Abteileinlass (123) mit dem hinteren Körpereinlass (106; 306; 406; 506) derart ausgerichtet ist, dass ein Luftströmungsweg durch das Abteil (121; 321; 421; 521) zu dem Mundstück (103; 303; 403; 503) gebildet ist, so dass das Abteil (121; 321; 421; 521) eine Zerstäubungskammer bildet, um die Inhalte des Abteils an das Mundstück (103; 303; 403; 503) abzugeben.

2. Trockenpulver-Inhalator nach Anspruch 1, **gekennzeichnet durch** die Tatsache, dass der hintere Abteileinlass (123) des oder jedes Pulverabteils so bemessen ist, dass im wesentlichen verhindert wird, dass das Pulver **durch** diesen aus dem Abteil (121; 321, 421; 521) aufgrund der Schwerkraft während des Befüllens austritt, und der hintere Abteileinlass wenigstens eine Abmessung hat, die höchstens 1 mm beträgt.

3. Trockenpulver-Inhalator nach Anspruch 2, **gekennzeichnet durch** die Tatsache, dass der oder jeder hintere Abteileinlass (123), der vorzugsweise die Gestalt eines Schlitzes annimmt, einen Trichter (150) bildet, der sich nach außen zu dem Abteil verjüngt.

4. Trockenpulver-Inhalator nach Anspruch 3, **gekennzeichnet durch** die Tatsache, dass der Verjüngungswinkel des Trichters (150) im Bereich zwischen 179° und dem Schüttwinkel des verwendeten pharmazeutischen Pulvers und vorzugsweise zwischen 120% und dem Schüttwinkel liegt.

5. Trockenpulver-Inhalator nach einem der vorhergehenden Ansprüche, **gekennzeichnet durch** die Tatsache, dass der Inhalatorkörper (100; 300; 400; 500) weiterhin Einrichtungen enthält, die eine Versperrung des Durchgangs von Luft durch die hinteren Abteileinlässe (123) und Körperseiteneinlässe (130; 330; 430; 530) **durch** den Benutzer verhindern.

6. Trockenpulver-Inhalator nach einem der vorhergehenden Ansprüche, **gekennzeichnet durch** die Tatsache, dass der oder jeder Behälter (110; 310; 410; 510) mit geringen Toleranzen in die Körperöffnung (102; 302; 402; 502) derart eingepasst ist, dass, wenn die vorderen und hinteren Abteileinlässe (120; 420; 520) des wenigstens einen Pulverabteils (121; 321; 421; 521) von den vorderen und hinteren Körpereinlässen (105; 305; 405; 505) versetzt sind, der Inhalatorkörper (100; 300; 400; 500) im wesentlichen dichtend mit den vorderen (120; 320; 420; 520) und den hinteren (123) Abteileinlässen in Eingriff steht, um so einen Austritt von Pulver aus dem Abteil zu verhindern.

7. Trockenpulver-Inhalator nach einem der vorhergehenden Ansprüche, **gekennzeichnet durch** die Tatsache, dass ein vorderer Körpereinlass (105; 305; 405; 505) an dem distalen Ende des Mundstückes (103; 303; 403; 503) (oder Nasenstück) vorgesehen ist, wobei der vordere Körpereinlass in Verbindung mit einem Pulverabteil (121; 321; 421; 521) einen Pulverzerstäubungsbereich bildet und den Luftstrom verringert, der das Pulverabteil verlässt und in das Mundstück eintritt.

8. Trockenpulver-Inhalator nach einem der vorhergehenden Ansprüche, **gekennzeichnet durch** die Tatsache, dass sich die Inhalatorkörperöffnung (102; 302) transversal zum Inhalatorkörper (100; 300) erstreckt und der Behälter (110; 310) gleitend entlang der Öffnung (102; 302) quer zum Inhalatorkörper (100; 300) beweglich ist, wobei die Längsachse des oder jedes Abteils (121; 321) parallel zur Längsachse des Mundstückes (103; 303) ausgerichtet ist.

9. Trockenpulver-Inhalator nach einem der Ansprüche 1 bis 7, **gekennzeichnet durch** die Tatsache, dass der Behälter (410; 510) mit der Öffnung (402; 502) drehbar in Eingriff bringbar ist.

10. Trockenpulver-Inhalator nach Anspruch 9, **gekennzeichnet durch** die Tatsache, dass der Behälter (410) zylinderförmig ist und sich in der Öffnung (402) um eine Achse (440) parallel zur Längsachse des Mundstückdurchgangs (403) dreht.

11. Trockenpulver-Inhalator nach Anspruch 9, **gekennzeichnet durch** die Tatsache, dass der Behälter (510) scheibenförmig ist und sich in der Öffnung (502) um eine Achse (540) dreht, die im wesentlichen senkrecht zur Längsachse des Mundstückdurchgangs (503) ist.

12. Trockenpulver-Inhalator nach einem der vorhergehenden Ansprüche, **gekennzeichnet durch** die Tatsache, dass bei Verwendung der Behälter (110; 310; 410; 510) nach vorne bewegt wird, um einen vorderen Abteileinlass (120; 420; 520) in Ausrichtung mit dem vorderen Körpereinlass (105; 305; 405; 505) **durch** eine einzige Bewegung des Behälters zu bringen.

13. Trockenpulver-Inhalator nach einem der vorhergehenden Ansprüche, **gekennzeichnet durch** die Tatsache, dass der Behälter (110; 310; 410; 510) wenigstens zwei Pulverabteile (121; 321; 421; 521) enthält, die voneinander getrennt sind.

14. Trockenpulver-Inhalator nach Anspruch 13, **gekennzeichnet durch** die Tatsache, dass er weiterhin Blockiereinrichtungen enthält, die in Funktion eine Bewegung des Behälters (310; 410; 510) in eine erste Richtung über den präzisen Punkt hinaus verhindern, an dem der erste vordere Abteileinlass (420; 520) mit dem vorderen Körpereinlass (305; 405; 505) ausgerichtet ist, und in ähnlicher Weise blockierend betätigt werden können, um eine Bewegung des Behälters in einer Richtung entgegengesetzt zu der ersten Richtung über den präzisen Punkt hinaus zu verhindern, an dem der zweite vordere Abteileinlass (420; 520) mit dem vorderen Körpereinlass ausgerichtet ist.

15. Trockenpulver-Inhalator nach einem der vorhergehenden Ansprüche, **gekennzeichnet durch** die Tatsache, dass er lediglich einen Behälter (110; 310; 410; 510) enthält, wobei sowohl der Inhalatorkörper (100; 300; 400; 500) als auch der Behälter als einheitliches Teil ausgebildet sind, so dass der Inhalator aus lediglich zwei Teilen besteht.

## Revendications

1. Inhalateur à poudre sèche adapté pour une distribution pulmonaire ou nasale, comprenant :
(a) un corps d'inhalateur (100, 300, 400, 500) ayant un embout (103, 303, 403, 503), une ouverture dans le corps (102, 302, 402, 502) et une entrée avant du corps (105, 305, 405, 505) s'étendant depuis l'embout vers l'ouverture dans le corps afin de permettre une communication fluidique ; et
(b) une cartouche (110, 310, 410, 510) montée de manière mobile dans l'ouverture dans le corps et ayant au moins un compartiment de poudre (121, 321, 421, 521) formé à l'intérieur qui comprend une entrée avant de compartiment (120, 420, 520) ; la cartouche étant mobile relativement au corps de l'inhalateur entre au moins une première position, dans laquelle l'entrée avant de compartiment (120, 420, 520) d'au moins un compartiment de poudre (121, 321, 421, 521) est décalée de l'entrée avant de corps (105, 305, 405, 505) de façon à isoler le contenu du compartiment de poudre de l'embout (103, 303, 403, 503), et une seconde position dans laquelle l'entrée avant du compartiment (120, 420, 520) d'au moins un compartiment de poudre (121, 321, 421, 521) est alignée avec l'entrée avant de corps (105, 305, 405, 505)
chacun parmi le corps d'inhalateur (100, 300, 400, 500) et la cartouche (110, 310, 410, 510) étant de construction unitaire, de sorte que l'inhalateur soit formé seulement de deux parties séparées qui sont mobiles l'une par rapport à l'autre ; et
la cartouche (110, 310, 410, 510) étant étroitement adaptée dans l'ouverture du corps (100, 300, 400, 500) de sorte que dans la première position relative de la cartouche (110, 310, 410, 510) , le corps (100, 300, 400, 500) se mette sensiblement en prise avec l'entrée avant (120, 420, 520), de façon à empêcher le contenu de s'en échapper ; **caractérisé en ce que**
le corps d'inhalateur (100, 300, 400, 500) comprend en outre une entrée arrière de corps (106, 306, 406) s'étendant à travers lui, vers l'ouverture dans le corps d'inhalateur et le ou chaque compartiment de poudre (121, 321, 421, 521) comprend en outre une entrée arrière de compartiment (123), dans laquelle, dans la première position de la cartouche (110, 310, 410, 510) relativement au corps (100, 300, 400, 500), le corps (100, 300, 400, 500) met sensiblement en prise de manière étanche l'entrée arrière de compartiment (123), de façon à empêcher, en cours d'utilisation, le contenu du compartiment (121, 321, 421, 521) de s'en échapper, et dans la seconde position de la cartouche (110, 310, 410, 510) relativement au corps (100, 300, 400, 500), l'entrée arrière de compartiment (123) s'aligne à l'entrée arrière du corps (106, 306, 406, 506) de façon à créer un chemin d'écoulement d'air à travers le compartiment (121, 321, 421, 521) jusqu'à l'embout (103, 303, 404, 503), de sorte que le compartiment (121, 321, 421, 521) forme une chambre de dispersion pour distribuer le contenu du compartiment à l'embout (103, 303, 403, 503).

2. Inhalateur à poudre sèche selon la revendication 1, **caractérisé par le fait que** l'entrée arrière de compartiment (123) du ou de chaque compartiment de poudre est dimensionnée, de façon à empêcher sensiblement la poudre de passer à travers, en dehors du compartiment (121, 321, 421, 521) sous la gravité pendant le remplissage, et ladite entrée arrière de compartiment a au moins une dimension de 1 mm ou moins.

3. Inhalateur à poudre sèche selon la revendication 2, **caractérisé par le fait que** l'entrée ou chaque entrée arrière de compartiment (123), qui prend de préférence la forme d'une fente, forme un entonnoir (150) se rétrécissant vers l'extérieur vers le compartiment.

4. Inhalateur à poudre sèche selon la revendication 3, **caractérisé par le fait que** l'angle de conicité de l'entonnoir (150) est compris entre 179° et l'angle de repos de la poudre pharmaceutique utilisée et de préférence entre 120% et ledit angle de repos.

5. Inhalateur à poudre sèche selon l'une quelconque des revendications précédentes, **caractérisé par le fait que** le corps d'inhalateur (100, 300, 400, 500) comprend en outre des moyens pour empêcher l'obstruction du passage d'air, par le biais d'entrées arrière de compartiment (123) et d'entrées latérales de corps (130, 330, 430, 530), par l'utilisateur.

6. Inhalateur à poudre sèche selon l'une quelconque des revendications précédentes, **caractérisé par le fait que** la ou chaque cartouche (110, 310, 410, 510) est étroitement adaptée dans l'ouverture dans le corps (102, 302, 402, 502) de sorte que, lorsque les entrées avant et arrière du compartiment (120, 420, 520) d'au moins un compartiment de poudre (121, 321, 421, 521) sont décalées des entrées avant et arrière du corps (105, 305, 405, 505), le corps d'inhalateur (100, 300, 400, 500) met en prise sensiblement de manière étanche les entrées de compartiment avant (120, 320, 420, 520) et arrière (123), de façon à empêcher que la poudre ne fuie dudit compartiment.

7. Inhalateur à poudre sèche selon l'une quelconque des revendications précédentes, **caractérisé par le fait qu'**une entrée avant de corps (105, 305, 405, 505) est fournie à l'extrémité distale de l'embout (103, 303, 403, 503) (ou embout nasal), ladite entrée avant de corps, combinée à un compartiment de poudre (121, 321, 421, 521), définit une zone de dispersion de poudre et réduit l'écoulement d'air qui quitte ledit compartiment de poudre et entre dans ledit embout.

8. Inhalateur à poudre sèche selon l'une quelconque des revendications précédentes, **caractérisé par le fait que** l'ouverture dans le corps d'inhalateur (102, 302) s'étend transversalement par rapport au corps d'inhalateur (100, 300) et la cartouche (110, 310) est mobile de manière coulissante le long de l'ouverture (102, 302) transversalement au corps d'inhalateur (100, 300), l'axe longitudinal de chaque ou du compartiment (121, 321) étant orienté parallèlement à l'axe longitudinal de l'embout (103, 303).

9. Inhalateur à poudre sèche selon l'une quelconque des revendications 1 à 7, **caractérisé par le fait que** la cartouche (410, 510) est mise en prise de manière rotative dans l'ouverture (402, 502).

10. Inhalateur à poudre sèche selon la revendication 9, **caractérisé par le fait que** la cartouche (410) est de forme cylindrique et tourne dans l'ouverture (402) autour d'un axe (440) parallèle à l'axe longitudinal du passage de l'embout (403).

11. Inhalateur à poudre sèche selon la revendication 9, **caractérisé par le fait que** la cartouche (510) est en forme de disque et tourne dans l'ouverture (502) autour d'un axe (540) sensiblement perpendiculaire à l'axe longitudinal du passage de l'embout (503).

12. Inhalateur à poudre sèche selon l'une quelconque des revendications précédentes, **caractérisé par le fait qu'**en cours d'utilisation, la cartouche (110, 310, 410, 510) est avancée pour mettre une entrée avant de compartiment (120, 420, 520) en alignement avec l'entrée avant du corps (105, 305, 405, 505) avec un seul mouvement de la cartouche.

13. Inhalateur à poudre sèche selon l'une quelconque des revendications précédentes, **caractérisé par le fait que** la cartouche (110, 310, 410, 510) comprend au moins deux compartiments de poudre (121, 321, 421, 521) qui sont isolés l'un de l'autre.

14. Inhalateur à poudre sèche selon la revendication 13, **caractérisé par le fait qu'**il comprend en outre un système de blocage actionnable pour empêcher le mouvement de la cartouche (310, 410, 510), dans une première direction au-delà du point précis où la première entrée avant de compartiment (420, 520) est en alignement avec l'entrée avant du corps (305, 405, 505) et un blocage similaire actionnable pour empêcher le mouvement de ladite cartouche, dans une direction contraire à la première direction au-delà du point précis où la seconde entrée avant de compartiment (420, 520) est en alignement avec ladite entrée avant du corps.

15. Inhalateur de poudre sèche selon l'une quelconque des revendications précédentes, **caractérisé par le fait qu'**il comprend juste une seule cartouche (110, 310, 410, 510), où chacun des éléments parmi le corps d'inhalateur (100, 300, 400, 500) et ladite cartouche est formée comme une partie unitaire, de sorte que l'inhalateur soit composé de juste deux parties.
